# EUROPEAN PATENT SPECIFICATION

(11) **EP 1 522 838 B1**
(45) Date of publication and mention of the grant of the patent: **22.09.2010**
(21) Application number: 04024179.6
(22) Date of filing: 11.10.2004
(51) Int. Cl.: G01M 3/22

(54) **Leak testing of hermetic enclosures for implantable energy storage devices**
Leck-Prüfung von hermetischen Gehäusen für implantierbare Energiespeichern-Vorrichtungen
Essai de l'étanchéité de boîtiers hermétiques pour dispositifs implantables de stockage d'énergie

(30) Priority: 10.10.2003 US 510601 P
(43) Date of publication of application: 13.04.2005
(73) Proprietor: Greatbatch Ltd., Clarence, NY 14031 (US)
(72) Inventor: Eberhard, Douglas P, Grand Island New York 14072 (US); Muffoletto, Barry C, Alden New York 14004 (US); Neff, Wolfram, Buffalo New York 14216 (US)
(74) Representative: Koepe, Gerd L.

(56) References cited:
- WO-A-90/13803
- WO-A-99/46572
- FR-A- 1 181 312
- FR-A- 2 710 747
- US-A- 2 486 199
- US-A- 3 672 207
- US-A- 4 948 683
- US-B1- 6 479 294
- SANKARAN V A ET AL: "Electrolytic capacitor life testing and prediction" INDUSTRY APPLICATIONS CONFERENCE, 1997. THIRTY-SECOND IAS ANNUAL MEETING, IAS '97., CONFERENCE RECORD OF THE 1997 IEEE NEW ORLEANS, LA, USA 5-9 OCT. 1997, NEW YORK, NY, USA,IEEE, US, vol. 2, 5 October 1997 (1997-10-05), pages 1058-1065, XP010248463 ISBN: 0-7803-4067-1

## Description

### BACKGROUND OF THE INVENTION

### 1. Field of the Invention

The present invention generally related to quality control of hermetic devices and, more particularly, to leak detection of sealed enclosures to ensure their hermeticity over several orders of magnitude, i.e., gross and fine leak detection. Confirming hermeticity is critical for any sealed enclosure, especially one housing of an electrical power source for an implantable medical device. The power source can be either an electrochemical cell or a capacitor.

In either case, the power source includes a negative electrode and a positive electrode physically segregated from each other by a separator and provided with an electrolyte. The specific chemistry of the cell or capacitor is not limited. For example, the cell can be of either a primary chemistry such as of a lithium/silver vanadium oxide or lithium/fluorinated carbon (CFx) couple or of a secondary chemistry such as a lithium ion cell and the capacitor could be a wet tantalum electrolytic type. The only requirement is that the hermetic enclosure for the power source has an electrolyte or some other liquid provided therein that has a vapor pressure more than about 13.3 Pa (1 mm Hg) for fine leak testing and about 0.133 Pa (0.01 mm Hg) for gross leak testing.

### 2. Prior Art

The document US-A 6,479,294 discloses a method and an apparatus for determining leaks in a sealed product container by immersing an allegedly leaking product into a liquid extraction medium which is capable of extracting a substance leaking from the sealed product and determining the substance leaked out from the sealed product in the extraction medium. Sealed products found not to leak liquid components fro within the sealed product may be subjected to a second gas leak test in an evacuated (empty) container into which the sealed product is placed, and analyzing the gas content of the evacuated container after a certain time for gases from within the sealed product which were present in the environment of the product before or at the time of sealing the product.
The document WO 90/13,803 discloses a method and apparatus for monitoring the presence of subsoil gases and liquids, and in the case of gases and/or liquids being present, for analyzing such gases and/or liquids in the environment of waste depositions for detecting environmental contamination.
The document WO 99/46,572 discloses a method and an apparatus for detecting leaks in hermetic packages. For a leak detection, the package (or container) containing a gas and/or a volatile liquid, which is used as an indicator (a possible indicator gas under vacuum) is placed in a vacuum chamber. In a second step, the vacuum chamber is purged with a purge gas. After purging, the vacuum chamber is doped with a calibrator gas. Then, the vacuum chamber is connected to a mass spectrometer. The mass spectrometer then detects both gases, calibrator gas and indicator gas, from the inside of the container being subject to leak detection, and the relative concentration measured serves as an indicator for an existence (or non-existence) of a leak.

The industry standard for testing the hermeticity of sealed enclosures is based on helium detection. In this test, the enclosure is placed in a bombing chamber pressurized with helium. A typical pressure is 6.895 bar (100 psi) and resident time is from one hour to several days. If a leak exists, helium is forced into the void volume of the enclosure. The time and pressure chosen depend on the leak size to be measured and the size of the void volume in the enclosure. After the prescribed time, the enclosure is removed from the bombing chamber and put in a vacuum leak detector where the presence of helium indicates a leak.

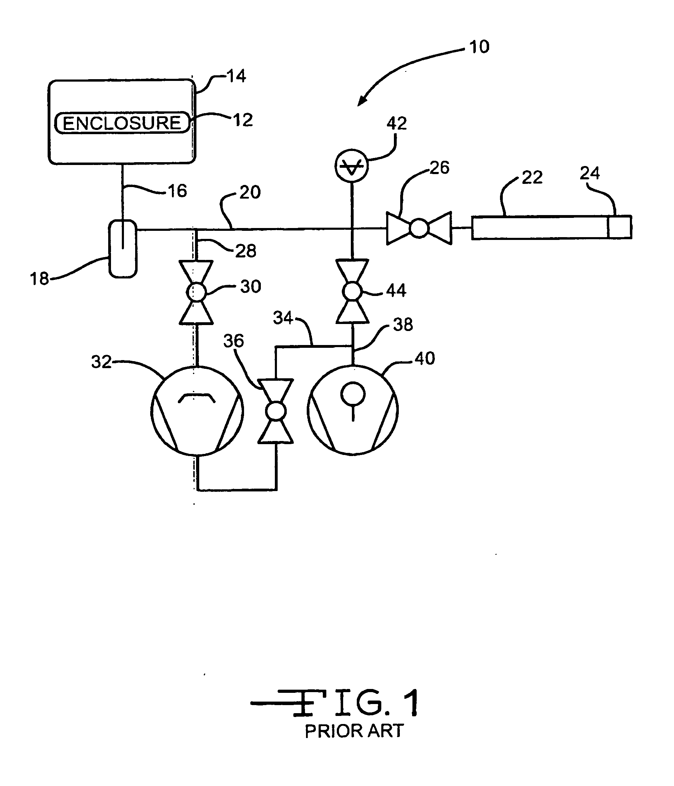

A fill port seal for an electrochemical cell predicated on this type of leak detection is described in U.S. Patent No. 6,203,937 to Kraska. Hollow glass bubbles residing between an inner press fit stainless steel ball and an outer metal cover serve as a getter absorbing helium that has leaked past the outer cover for later detection. However, the Kraska seal structure has several shortcomings. Not only is leak detection sensitive to the ratio of leak size to void volume, the detection apparatus 10, as schematically illustrated in Fig. 1, is rather complex.

To detect the presence of helium in the getter, the cell as a sealed enclosure 12 is then loaded into the vacuum chamber 14 of the detection apparatus 10. A conduit 16 connects the chamber 14 to a cold trap 18. The use of a cold trap is essential to remove water vapor or other condensable gases in the vacuum system that could impair proper operation.

Conduit 20 leaving the trap 18 leads to a mass spectrometer 22 connected to an analyzer 24. A valve 26 is in the conduit 20. Upstream from valve 26 is a conduit 28 leading to a valve 30 and a high vacuum pump 32. A conduit 34 with valve 36 makes a T-connection with conduit 38. The ends of this conduit 38 lead to a roughing pump 40 and connect back into the conduit 20 upstream from valve 26. A pressure gauge 42 ties into the junction of conduits 20 and 38. A valve 44 is located between the roughing pump 40 and conduit 20.

A helium leak detection test begins by placing the sealed enclosure 12 in the vacuum chamber 14. Valves 26, 30 and 44 are closed with valve 36 between the high vacuum pump 32 and the roughing pump 40 being open. Valve 36 is open so exhaust from the high vacuum pump 32 can be remove-ed. The vacuum chamber 14 is closed, valve 36 is closed and valve 44 is opened. This provides for communication between the roughing pump 40 and the vacuum chamber 14 through trap 18. Roughing pump 40 reduces the pressure inside the chamber 14 to about 0.13 mbar (100 mTorr). When gauge 42 indicates a system vacuum of about 0.13 mbar (100 mTorr), valve 44 is closed and valves 30 and 36 are opened bringing the high vacuum pump 32 into the system. The high vacuum pump 32 in communication with the vacuum chamber 14 through the nitrogen trap 18 reduces the system pressure to about 1.3 mbar (1x 10⁻⁶ Torr). Suitable pumps for this purpose include oil diffusion pumps, and turbo or cryogenic pumps. The cold trap 18 removes any residual moisture from the atmosphere evacuated from the vacuum chamber 14 so that the pumps 32 and 40 are not damaged once they are put on-line.

When the test condition vacuum is reached, the analyzer system consisting of the mass spectrometer 22 and analyzer 24 is connected to the vacuum chamber 14 by opening valve 26. Typically, a quadrupol mass spectrometer is used because of its ability to selectively pass particles with a characteristic specific charge. The analyzer 24 detects and displays the amount of helium that passes through the mass spectrometer tuned to helium. A similar system is described in US-A 2,486,199.

Helium leak detection using this type of system has several shortcomings. First, the method only works for enclosures that have a void volume, which is the case for most electronic components, but enclosures such as those for batteries and wet tantalum capacitors are completely filled with a liquid electrolyte. For them, the test does not work reliably. Secondly, setup parameters for a helium leak detection test are dependant on the ratio of leak size to void volume. Since the leak size of a sealed enclosure is not necessarily known, and can vary by several orders of magnitude from one enclosure to the next, correct production setup poses a challenge. Further, the equipment for a helium leak detection is rather complex. For example, vacuum parts need constant maintenance. Gross leaks can contaminate the system to the point that parts have to be replaced, leading to downtime. Gross leaks also decreased system sensitivity, which may not be recognized until the next calibration check. Therefore, a separate test to identify gross leaks is typically first used in conjunction with this test. The mass spectrometer 22 needs constant verification and typically drifts with time and temperature. Consequently, adjustment of a helium leak detector system before use on every shift using a calibrated leak is not uncommon. Finally, a part failure, such as a stuck valve, often leads to downtime and damage to system components.

These problems are avoided when the existence of a leak in a sealed enclosure is based on detecting compounds with a relatively high vapor pressure present in a liquid or added to the liquid in the enclosure for the purpose of detection. In either case, the enclosure is placed in a test chamber and the air therein is analyzed for the detectable compounds. Examples of such a test unit include, but are not limited to, mass spectrometers, chromatographic methods and time-of flight or ion mobility testing.

### SUMMARY OF THE INVENTION

Implantable medical devices such as cardiac pacemakers, drug pumps, neurostimulators include at least one electrochemical cell as a power source. Defibrillators also include at least one capacitor. It is important to ensure that these power sources are hermetically sealed. This is because they contain many caustic and harmful materials. Should any of them escape from the enclosure housing, the escaping materials could not only harm the device itself, but they could prove lethal. Therefore, it is critically important to be able to quickly, but accurately test sealed enclosures housing implantable medical components to ensure their hermeticity.

Sensing the presence of vapors escaping from contents housed in the enclosures, such as vapors from escaping electrolyte materials does this. This present invention sensing technique replaces the older method of helium detection and utilizes compounds having a relatively high vapor pressure present in the liquid or added to the liquid contained in the enclosure. The enclosure is then placed in an appropriate test chamber and the air therein is analyzed for the specific compounds by standard analytical techniques including mass spectrometry, chromatography and time-of-flight testing.
The present invention relates to a method for determining the sealed integrity of an enclosure, comprising the steps of:
a) providing, in a first chamber sized to contain the enclosure, the enclosure comprising at least a first part secured to a second part with a liquid contained in at least a portion of an enclosed volume of the enclosure;
b) providing the liquid comprising at least one compound constituent having a vapor pressure at 25°C of more than 13.3 Pa;
c) flowing a gaseous stream of purified air or ambient air past the enclosure in the first chamber thereby providing an analyte; and
d) analyzing the analyte for the presence of the compound indicating that the enclosure is leaking or for the absence of the compound indicating the enclosure is hermetically sealed.
These and other objects of the present invention will become increasingly more apparent to those skilled in the art by a reading of the following detailed description in conjunction with the appended drawings.

### BRIEF DESCRIPTION OF THE DRAWINGS

Fig. 1 is a schematic view of a conventional helium leak detection apparatus 10.
Fig. 2 is a schematic illustration of an ion mobility leak detection analyzer 100 according to the present invention.
Fig. 3 is a graph of the vapor pressure of acetic acid versus temperature.
Fig. 4 is a graph of the detecting sensitivity of acetic acid versus sample air flow.
Fig. 5 is a schematic of a continuous ion mobility analyzer 200 according to the present invention.
Fig. 6 is a schematic illustration of a batch ion mobility leak detection analyzer 300 operational at ambient.
Fig. 7 is a schematic illustration of a batch ion mobility leak detection analyzer 320 operating under vacuum.
Fig. 8 is a schematic representation of the pressures inside and outside a leaking enclosure.

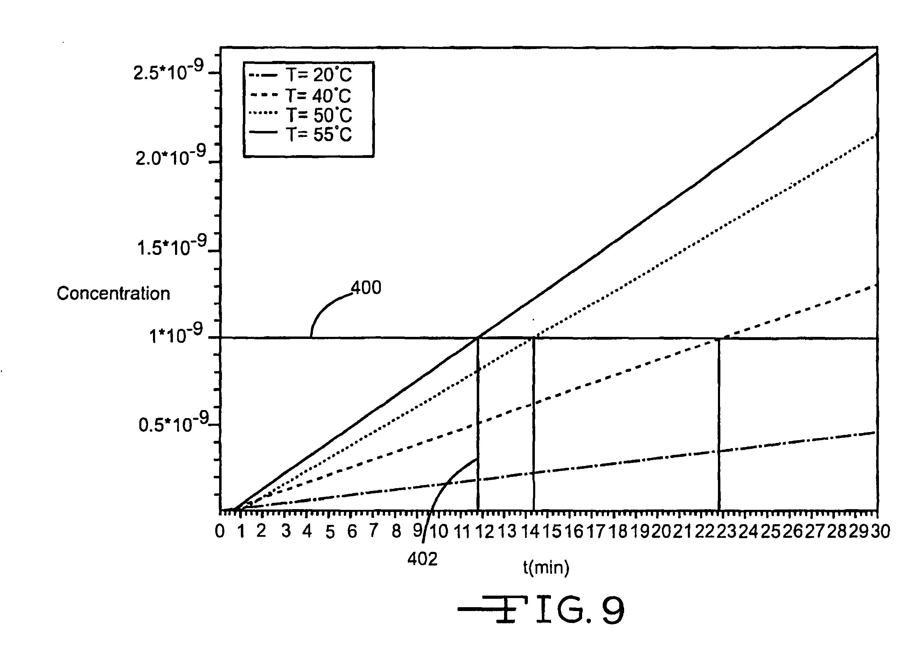

Figure 9 is a graph of the concentration of acetic acid vapor in a 1.2-liter test chamber accumulated by vaporizing a liquid containing 23% acetic acid and deionized water streaming through leaks of a device with a leak rate of 1 x 10⁻⁷ std. atm. cc/sec, corresponding to 1.01.10⁻² Pa·cm³/s.

### DETAILED DESCRIPTION OF THE PREFERRED EMBODIMENTS

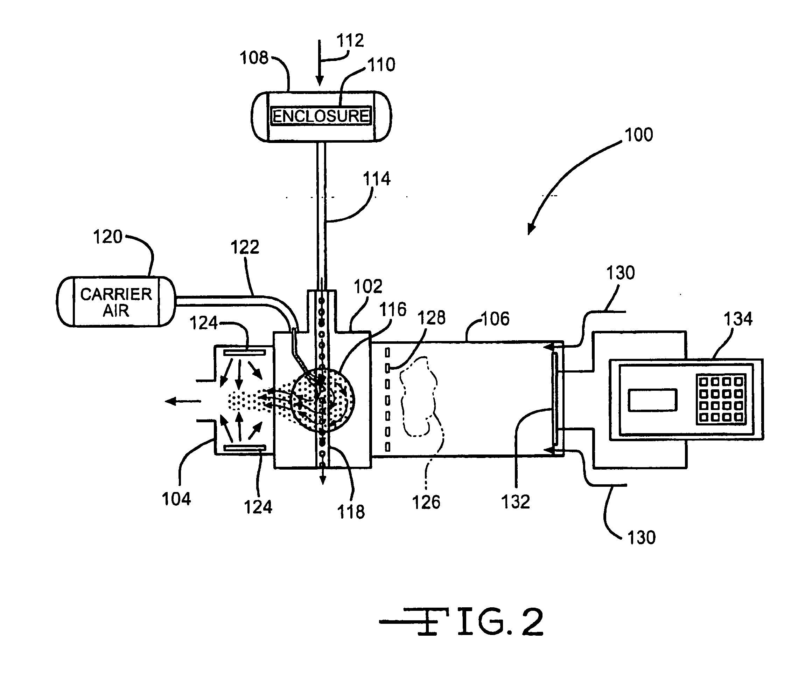

Referring back to the drawings, Fig. 2 schematically illustrates an ion mobility detection analyzer 100 according to the present invention. The analyzer 100 comprises a reaction chamber having a main section 102, a reaction region 104, a drift tube 106 and an inlet chamber 108 containing the sealed enclosure 110. Ambient air 112 is drawn into the inlet chamber 108 past the sealed enclosure 110 through a conduit 114 and into the main section 102 of the reaction chamber. There, an internal eductor 116 causes the ambient air sample to flow over a semi-permeable membrane 118, which provides various levels of sensitivity based on permeation rates. Molecules of the compound of interest picked up by the ambient air flowing over the sealed enclosure 110 permeate through the membrane 118 and are picked up by a purified carrier airflow 120 entering the main section 102 through conduit 122 to sweep the opposite side of the membrane 118. The carrier airflow 120 delivers the air sample molecules to the reaction region 104 containing a small Ni⁶³ radioactive source 124. There, the air sample is ionized as a result of a series of ion-molecule reactions. Dopant compounds added to the carrier stream enter into the ion-molecule chain of reactions and provide a degree of selectivity based on the charge affinity of the analyte. Once the air sample is ionized, the ions begin to drift 126 towards the drift tube 106 at the opposite end of the reaction chamber due to the influence of an electrostatic field.

A shutter grid 128 is located at the entrance to the drift tube 106. The shutter grid 128, which is biased electrically to either block the ions, or allow them to pass through, is pulsed periodically to allow the ions into the drift tube 106. There, they begin to separate out based on their size and shape while flowing counter to a drift gas flow 130 introduced at the end of the drift tube 106. A detector plate 132 located at the far end of drift tube 106 detects the arrival of the ions by producing a current. Smaller ions move faster through the drift tube than larger ones and arrive at the detector plate 132 first. Amplified current from the detector is measured as a function of time and a spectrum is then generated. A microprocessor 134 evaluates the spectrum for the target compound, and determines its concentration based on the peak spectrum height. Because specificity of the membrane 118 enhances ionization and time-of-flight, this system offers a relatively high degree of certainty that the analyzer 100 is measuring only the compound of interest, even in the presence of other interferents. An ion mobility tester similar to that described with respect to the analyzer 100 shown in Fig. 2 is commercially available from Molecular Analytics, Inc., Boulder, Colorado.

For example, a wet tantalum capacitor has an electrolyte containing acetic acid. This compound has a relatively high vapor pressure and is ideal for leak detection using an ion mobility detector analyzer. To further enhance the vaporization rate of acetic acid, the capacitors are heated to a temperature of about 125°C, 54°C to 55°C being preferred.

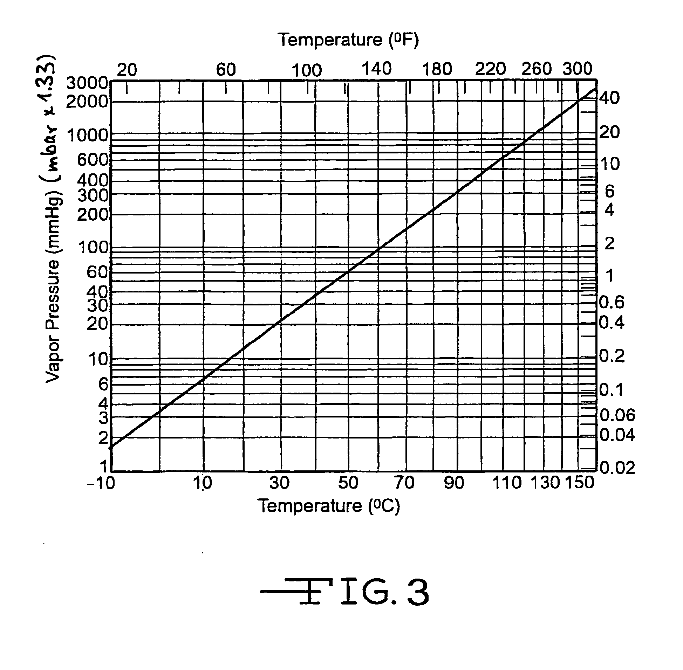

A typical wet tantalum capacitor manufacture by Wilson Greatbatch Technologies, Inc., Clarence, New York has an electrolyte volume of about 0.72 cm³. Acetic acid is typically present in the electrolyte of this model capacitor in a concentration of about 15%, by weight. The vapor pressure of acetic acid is shown in Fig. 3. The vapor pressure of acetic acid is relatively high, resulting in complete vaporization of small quantities of acetic acid.

Assume that within a time frame of 10 years, it is desirable to lose no more than 5% of this volume, or about 0.0375 cm³. Since the electrolyte is mostly water, it is assumed that the density of the electrolyte is 1 g/cm³. The amount of acetic acid in a cubic centimeter of electrolyte is calculated as 0.0375 cm³ x 1 g/cm³ = 0.035 grams. There are 5.26 x 10⁶ minutes in a 10-year period. Losing 0.035 grams of electrolyte over ten years equates to a leak rate of 6.7 x 10⁻⁹ grams of electrolyte/minute over the ten-year period. The fraction of acetic acid in the electrolyte is 15%, by volume. Therefore, the leak detection rate is 1 x 10⁻⁹ grams of acetic acid/minute.

The molecular weight of acetic acid (C₂H₄O₂) is 2(12) + 4(1) + 2(16) = 60 grams/mole. This compound has a specific volume of 22.4 liters/mol, divided by 60 g/mol = 0.373 1/gram. This results in a vapor volume of 3.73 x 10⁻¹⁰ liters/minute = 3.73 x 10⁻⁷ cm³/minute.

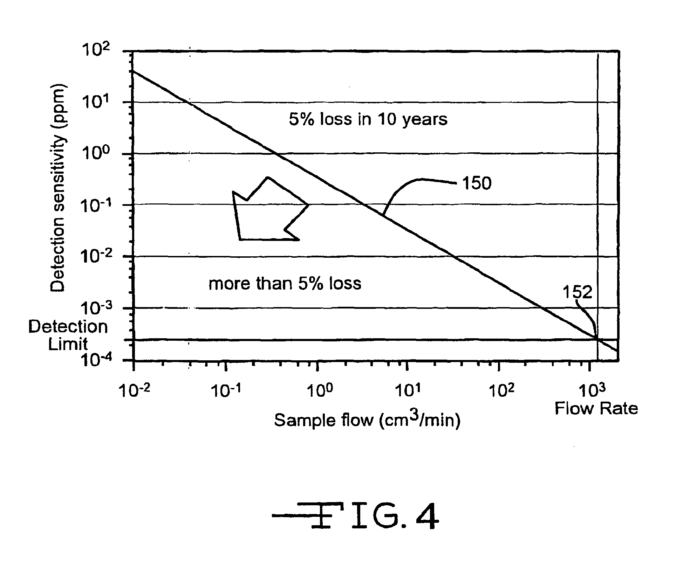

In a typical test application, clean air constantly streams at a set flow rate over the device. The vapor escaping through leaks in the device mixes with that air with the acetic acid being present at a low concentration. Using a device that samples air at a constant rate, for example, an ion mobility detector requires a specific sensitivity to detect this leak rate. A graphical representation of the requirement based on sample airflow is shown in Fig. 4. The sensitivity of an ion mobility leak detection analyzer 100 as previously described with respect to Fig. 2 and commercially available from Molecular Analytics, Inc., Boulder, Colorado was demonstrated to be 0.25 ppb at a flow rate of 1.2 1/min. This is indicated in the graph of Fig. 3 for acetic acid as line 150. Acetic acid has a vapor pressure of about 15.96 mbar (12 Torr (mm Hg) ) at 20°C. Assuming a sample flow rate of 10³ cm³/min (1 liters/min) of purified air, a limit of 0.3 ppb is needed to detect acetic acid, as indicated by the point labeled 152 on the graph. This is well within the capability of an ion mobility detector. Thus, an ion mobility leak detection analyzer 100 is capable of identifying a capacitor that leaks 5% over 10 years even in this very simple setting. In comparison, water has a vapor pressure of about 23.94 mbar (18 Torr) at 20 °C.

Since an ion mobility system is capable of operation at ambient pressure, different modes of operation based on expected leak rates and the vapor pressure of the target compound are feasible. This includes operating in a continuous mode for high leak rates or for compounds with a relatively high vapor pressure. A more sensitive method is to operate in batch mode at ambient pressure in the test chamber. An even more reliable technique is to operate in batch mode with the enclosures being subjected to a vacuum to detect both fine and gross leaks.

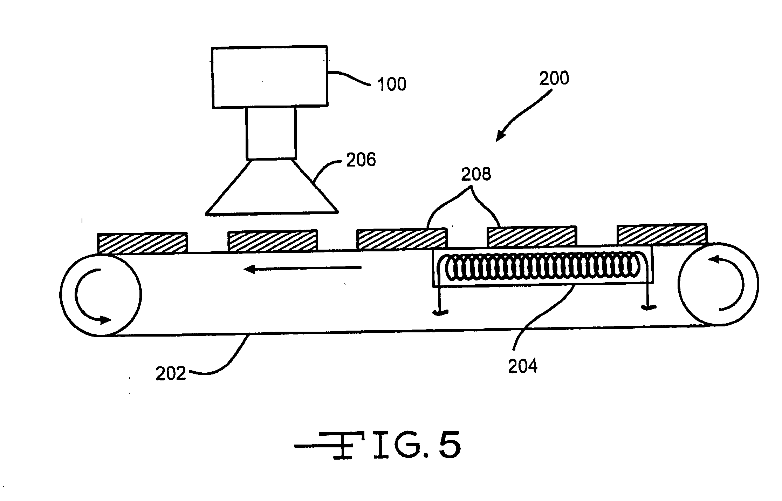

A schematic of a continuously operated ion mobility detector analyzer 200 is shown in Fig. 5. The continuous analyzer 200 consists of a conveyor belt 202 provided with a heater 204 to increase testing sensitivity. A sniffer 206 samples the air in the immediate vicinity of the enclosures 208. This air is moved to the ion mobility detector analyzer 100 previously described with respect to Fig. 2. A continuous ion mobility system is very effective because it is a high throughput operation that is well suited for gross leaks or detection of enclosures that contain liquid compounds with relatively high vapor pressures.

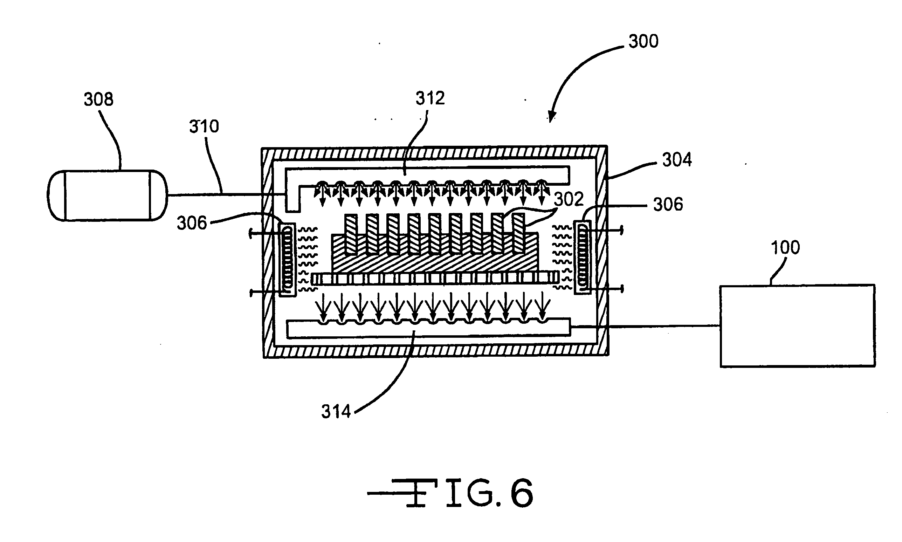

A schematic of a batch ion mobility analyzer 300 is illustrated in Fig. 6. The batch analyzer 300 is operational at ambient pressure with one or a plurality of sealed enclosures 302 residing in a sample chamber 304. If desired, the enclosures 302 are heated with heaters 306. The air supplied to the chamber 304 is ambient air, purified air or another suitable gas from a container 308. In any event, air from the container 308 travels along a conduit 310 and into the sample chamber 304 through a manifold 312 that allows uniform air flow over the sealed enclosures 302. After flowing over the enclosures, the airflow moves to a collector 314 where it is sampled by the ion mobility detector analyzer 100 previously described with respect to Fig. 2.

If the analyzer 300 detects the presence of the relatively high vapor pressure compound of interest, it is not known which one of the enclosures 302 is leaking. This means that the test must be re-run with a partial lot until the "leaker" is identified. The main advantage of this system is that it uses purifier air or specialized gases that minimize background effects present in ambient air.

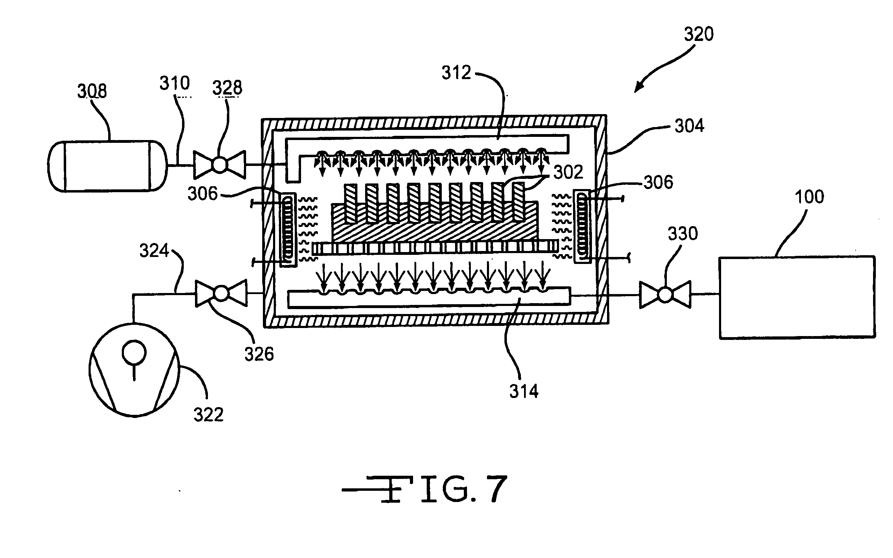

A schematic of an ion mobility analyzer in a batch mode where the sealed enclosures 302 are subjected to vacuum is shown in Fig. 7. This analyzer 320 is essentially the same as the ambient batch analyzer 300 of Fig. 6 with the addition of a vacuum pump 322 connected to the test chamber 304 by a conduit 324. The sealed enclosures 302 are loaded into the test chamber 304 and a vacuum is drawn on the chamber by pump 322. A simple roughing pump is sufficient, however, a high vacuum pump is not necessary. When a specified pressure is reached, valve 326 is closed. This is done to avoid pumping out gaseous components that may leak from the enclosures. The enclosures are "soaked" in vacuum for a specified time (dependent on detection limit and vapor pressure). During this time, acetic acid vapors are allowed to accumulate in the test chamber 304. Then, valve 328 is opened to bring the test chamber 304 to atmospheric pressure and valve 330 is opened to allow the gas mix in the chamber to be sampled by the ion mobility tester 100.

The vacuum batch method offers, in addition to the increased sensitivity, the advantage of allowing a direct correlation between the standard leak rate (L) as defined in MIL-STD-883, Method 1014, and the concentration of the electrolyte component with a relatively high vapor pressure in a chamber of a given size and at a given temperature. MIL-STD-883, Method 1014 defines the standard leak rate as that quantity of dry air at 25 °C in atmospheric cubic centimeters flowing through a leak or multiple leak paths per second when the high pressure side is at 1.013 bar (1 atmosphere (760 mm Hg absolute) ) and the low pressure side is at a pressure not greater than 1.33 mbar (1 mm Hg absolute). Standard leak rate should be expressed in units of atmosphere cubic centimeters per second (atm cc/sec.).

The vacuum batch ion mobility method allows for a direct comparison of the electrolyte vapor flow rate with the flow rate of air. The main differences are that while the pressure difference driving the air through the leak is between 1.009 bar (759 mm Hg) and 1.010 bar (760 mm Hg) (dependent on the quality of the vacuum), the pressure across the leak in the vacuum batch detection method is that of the vapor pressure of the electrolyte. Further only the chemical component that the detector is tuned for is usable for the detection. This is a fraction of the total vapor getting through the leak. Also, the different gas flow parameters like gas viscosity have to be considered when making the comparison.

The volume of the detected component that is accumulated inside the test chamber can be calculated by multiplying the flow rate of the detected component by the soak time the enclosure (capacitor or cell) resides in the chamber 108. The accumulated vapor is then diluted when the chamber 108 is backfilled with clean, dry air. The concentration of the vapor component can be calculated by dividing the accumulated vapor volume by the total chamber volume. When the gas is pushed into the detector, this concentration is measured as the peak value.

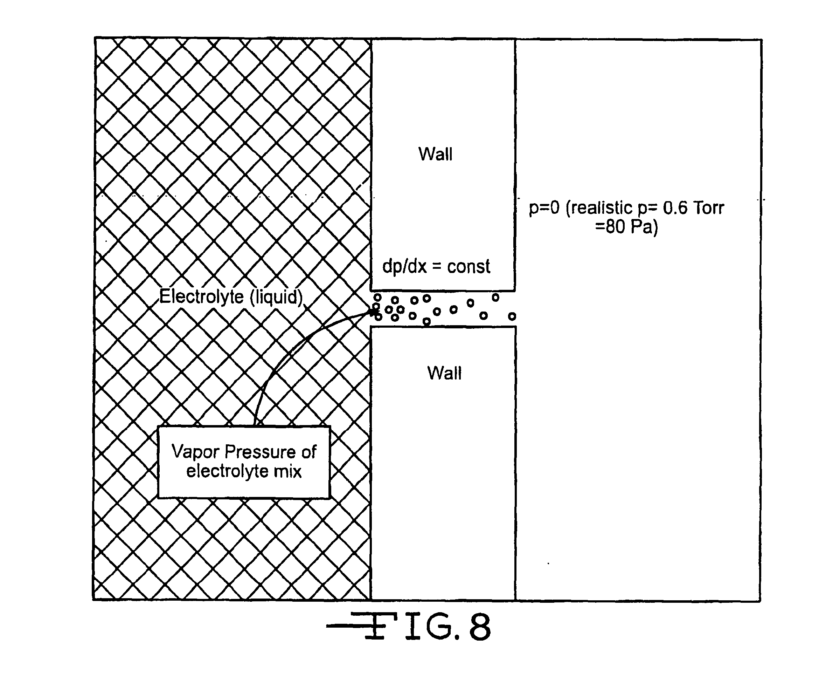

As shown in Fig. 8, an example is the calculation of the concentration of 23% acetic acid in deionized water flowing through a leak with a standard leak rate or 1.01·10⁻²Pa·cm³/s (1 x 10⁻⁷ std. atm. cc/sec.) into a 1.2 liter test chamber. The calculation is done for four different temperatures. The horizontal line 400 in the graph indicates the value where the ion mobility tester on this setting can differentiate a true trace amount of acetic acid from background fluctuations. This value is highly dependent on the design of the chamber and the piping system. The graph is to be read the following manner: if the test chamber temperature is at 55°C, and an enclosure (capacitor or cell) is held in vacuum for at least 11 minutes and 15 seconds (line 402), an acetic acid concentration of higher than 1 ppb (1 x 10⁻⁹) indicates a leak larger than 1.01·10⁻² Pa·cm³/s (1 x 10⁻⁷ std. atm. cc/sec).

From the graph in Fig. 8, it is apparent that the test time is dependent on the test temperature. In general, a higher test temperature increases the vapor pressure inside the enclosure and makes the test more sensitive, allowing for shorter test times. The highest temperature where the test can be performed is determined by the temperature limit of the device being tested.

Another present invention leak detection method relies on a mass spectrometer to test for a specific chemical leaking from a liquid in a sealed enclosure. The mass spectrometer method is in principle similar to the conventional helium leak detection system described in Fig. 1. Instead of detecting helium introduced as a foreign material into a potential leak in the enclosure, however, the mass spectrometer is adjusted to detect a particular compound known to be present in a liquid contained in the enclosure, for example a compound of an electrolyte in an electrochemical cell or capacitor. The testing cycle begins by placing the sealed enclosure in a vacuum chamber evacuated to a pressure low enough to enable the mass spectrometer to sample the chamber air. If the mass spectrometer detects the compound of interest at a level above a specified threshold, the test enclosure is deemed a leaker.

Another embodiment of a leak detection method according to the present invention relies on a chromatographic system. The two most common chromatographic methods are gas and liquid chromatography. For gas chromatography, the atmosphere around the enclosure is sampled and brought into contact with a medium that allows the air to diffuse into it. Different compounds diffuse at different speeds, leading to separation of the compounds in the air. In liquid chromatography, the sample is immersed in a liquid that serves as a medium through which the leaking compounds diffuse.

Beside acetic acid, other liquid components typically present in capacitor and electrochemical cell electrolytes that are detectible according to the present invention include ethylene glycol, diethylene glycol, propylene glycol, dipropylene glycol, glycerol, 2-methyl-1,3-propandoil, tetraethylene glycol, polyethylene glycols, polypropylene glycols, polyethylene polypropylene glycol copolymers, ethylene glycol methyl ether, ethylene glycol ethyl ether, diethylene glycol methyl ether, diethylene glycol ethyl ether, dipropylene glycol methyl ether, tripropylene glycol methyl ether, ethylene glycol dimethyl ether, triethylene glycol dimethyl ether, N-ethylformamide, N-methylformamide, N,N-dimethylformamide, N-methylacetamide, N,N-dimethylacetamide, N-methylpyrrolidone, dimethyl acetamide, ethyl lactate, ethylene diacetate, acetonitrile, propionitrile, methoxypropionitrile, gamma-butyrolatone, gamma-valerolactone, dimethyl carbonate, diethyl carbonate, dipropyl carbonate, ethylene carbonate, propylene carbonate, butylene carbonate, ethyl methyl carbonate, methyl propyl carbonate, ethyl propyl carbonate, iso-propyl methyl carbonate, sulfolane, 3-methylsulfolane, dimethyl sulfoxide, dimethyl formamide, dimethyl acetate, dimethylsulfolane, tetrahydrofuran, methyl acetate, diglyme, triglyme, tetraglyme, diisopropylether, 1,2-dimethoxyethane, 1,2-diethoxyethane, 1-ethoxy,2-methoxyethane, 2-methyltetrahydrofuran, 3-methyl-2-oxazolidinone, benzene, cumene, ethyl benzene, ethyldiglyme, ethylmonoglyme, fluorotrichloromethane, methylene chloride, propylsulfone, pseudocumene, tetraethylorthosilicate, toluene, m-xylene, o-xylene, ammonium acetate, ammonium phosphate, ammonium borate, propionic acid, butyric acid, methylbutyric acid, iso-butyric acid, trimethylacetic acid, and mixtures thereof.

Thus, the present invention has been particularly described with respect to a sealed enclosure being either a capacitor or an electrochemical cell. However, it will be apparent to those skilled in the art that the present leak detection methods are equally applicable to any sealed enclosure having a first part sealed to a second part with a liquid contained therein. The liquid need not occupy the entire volume of the enclosure, but must contain at least one component having a vapor pressure at 25 °C of more than about 1.33 Pa (0.1 mm Hg). This component can assist in the functioning of the device such as an electrolyte, or be added for the sole purpose of leak detection.
It is appreciated that various modifications to the inventive concepts described herein may be apparent to those of ordinary skill in the art without departing from the scope of the present invention as defined by the appended claims.

## Claims

1. A method of determining the sealed integrity of an enclosure (110), comprising the steps of:
a) providing, in a first chamber sized to contain the enclosure (110), the enclosure (110) comprising at least a first part secured to a second part with a liquid contained in at least a portion of an enclosed volume of the enclosure (110);
b) providing the liquid comprising at least one compound constituent having a vapor pressure at 25°C of more than 13.3 Pa;
c) flowing a gaseous stream of purified air or ambient air past the enclosure (110) in the first chamber thereby providing an analyte; and
d) analyzing the analyte for the presence of the compound indicating that the enclosure (110) is leaking or for the absence of the compound indicating the enclosure (110) is hermetically sealed.

2. The method of claim 1, wherein the compound is selected from the group consisting of acetic acid, ethylene glycol, diethylene glycol, propylene glycol, dipropylene glycol, glycerol, 2-methyl-1,3-propandiol, tetraethylene glycol, polyethylene glycols, polypropylene glycols, polyethylene polypropylene glycol copolymers, ethylene glycol methyl ether, ethylene glycol ethyl ether, diethylene glycol methyl ether, diethylene glycol ethyl ether, dipropylene glycol methyl ether, tripropylene glycol methyl ether, ethylene glycol dimethyl ether, triethylene glycol dimethyl ether, N-ethylformamide, N-methylformamide, N,N-dimethylformamide, N-methylacetamide, N,N-dimethylacetamide, N-methylpyrrolidone, dimethyl acetamide, ethyl lactate, ethylene diacetate, acetonitrile, propionitrile, methoxypropionitrile, γ-butyrolactone, γ-valerolactone, dimethyl carbonate, diethyl carbonate, dipropyl carbonate, ethylene carbonate, propylene carbonate, butylene carbonate, ethyl methyl carbonate, methyl propyl carbonate, ethyl propyl carbonate, isopropyl methyl carbonate, sulfolane, 3-methylsulfolane, dimethyl sulfoxide, dimethyl formamide, dimethyl acetate, dimethylsulfolane, tetrahydrofuran, methyl acetate, diglyme, triglyme, tetraglyme, diisopropylether, 1,2-dimethoxyethane, 1,2-diethoxyethane, 1-ethoxy-2-methoxyethane, 2-methyltetrahydrofuran, 3-methyl-2-oxazolidinone, benzene, cumene, ethyl benzene, ethyldiglyme, ethylmonoglyme, fluorotrichloromethane, methylene chloride, propylsulfone, pseudocumene, tetraethylorthosilicate, toluene, m-xylene, oxylene, ammonium acetate, ammonium phosphate, ammonium borate, propionic acid, butyric acid, methylbutyric acid, iso-butyric acid, trimethylacetic acid, and mixtures thereof.

3. The method of claim 1 or claim 2, wherein the liquid comprises an electrolyte of an electrochemical cell or capacitor, preferably wherein the liquid comprises an electrolyte, and the compound is acetic acid.

4. The method of any of claims 1 to 3, including analyzing the analyte by one of the group consisting of an ion mobility detection analyzer, a mass spectrometer, and a chromatographer.

5. The method of any of claims 1 to 4, including using an ion mobility detector to analyze the analyte for the presence of the compound indicating that the enclosure is leaking or for the absence of the compound indicating the enclosure is hermetically sealed, preferably including analyzing the analyte using an ion mobility detection analyzer at ambient pressure, or including analyzing the analyte using an ion mobility detection analyzer under vacuum, more preferably including using the ion mobility detection analyzer in a continuous mode or a batch mode.

6. The method of any of claims 1 to 5, wherein the hermetically sealed enclosure (110) comprises either an electrochemical cell or a capacitor, and further including incorporating the hermetically sealed electrochemical cell or capacitor into an implantable medical device as its power source.

7. The method of any of claims 1 to 6, including heating the enclosure (110) to a temperature up to 125°C as the gaseous stream is flown past it.

## Patentansprüche

1. Verfahren zur Bestimmung der dichten Unversehrtheit eines Gehäuses (110), umfassend die Schritte
a) Bereitstellen des Gehäuses (110), das wenigstens einen ersten Teil, der an einem zweiten Teil befestigt ist, mit einer wenigstens in einem Bereich eines eingeschlossenen Volumens des Gehäuses (110) enthaltenen Flüssigkeit umfasst, in einer ersten Kammer, die größenmäßig so angepasst ist, dass sie das Gehäuse (110) enthält;
b) Bereitstellen der Flüssigkeit, die wenigstens einen Verbindungs-Bestandteil umfasst, der einen Dampfdruck bei 25 °C von mehr als 13,3 Pa aufweist;
c) Strömenlassen eines gasförmigen Stroms gereinigter Luft oder Umgebungsluft entlang dem Gehäuse (110) in der ersten Kammer, wodurch man eine zu analysierende Substanz bereitstellt; und
d) Analysieren der zu analysierenden Substanz auf die Gegenwart der Verbindung, was anzeigt, dass das Gehäuse (110) ein Leck aufweist, oder auf die Abwesenheit der Verbindung, was anzeigt, dass das Gehäuse (110) hermetisch dicht ist.

2. Verfahren nach Anspruch 1, worin die Verbindung gewählt ist aus der Gruppe, die besteht aus Essigsäure, Ethylenglycol, Diethylenglycol, Propylenglycol, Dipropylenglycol, Glycerol, 2-Methyl-1,3-propandiol, Tetraethylenglycol, Polyethylenglycole, Polypropylenglycole, Polyethylenpolypropylenglycol-Copolymere, Ethylenglycolmethylether, Ethylenglycolethylether, Diethylenglycolmethylether, Diethylenglycolethylether, Dipropylenglycolmethylether, Tripropylenglycolmethylether, Ethylenglycoldimethylether, Triethylenglycoldimethylether, N-Ethylformamid, N-Methylformamid, N,N-Dimethylformamid, N-Methylacetamid, N,N-Dimethylacetamid, N-Methylpyrrolidon, Dimethylacetamid, Ethyllactat, Ethylendiacetat, Acetonitril, Propionitril, Methoxypropionitril, γ-Butyrolacton, γ-Valerolacton, Dimethylcarbonat, Diethylcarbonat, Dipropylcarbonat, Ethylencarbonat, Propylencarbonat, Butylencarbonat, Ethylmethylcarbonat, Methylpropylcarbonat, Ethylpropylcarbonat, Isopropylmethylcarbonat, Sulfolan, 3-Methylsulfolan, Dimethylsulfoxid, Dimethylformamid, Dimethylacetat, Dimethylsulfolan, Tetrahydrofuran, Methylacetat, Diglyme, Triglyme, Tetraglyme, Diisopropylether, 1,2-Dimethoxyethan, 1,2-Diethoxyethan, 1-Ethoxy-2-methoxyethan, 2-Methyltetrahydrofuran, 3-Methyl-2-oxazolidinon, Benzol, Cumol, Ethylbenzol, Ethyldiglyme, Ethylmonoglyme, Fluortrichlormethan, Methylenchlorid, Propylsulfon, Pseudocumol, Tetraethylorthosilicat, Toluol, m-Xylol, o-Xylol, Ammoniumacetat, Ammoniumphosphat, Ammoniumborat, Propionsäure, Buttersäure, Methylbuttersäure, Isobuttersäure, Trimethylessigsäure und Mischungen daraus.

3. Verfahren nach Anspruch 1 oder Anspruch 2, worin die Flüssigkeit einen Elektrolyten einer elektrochemischen Zelle oder eines Kondensators umfasst, vorzugsweise worin die Flüssigkeit einen Elektrolyten umfasst, und die Verbindung Essigsäure ist.

4. Verfahren nach irgendeinem der Ansprüche 1 bis 3, einschließend ein Analysieren der zu analysierenden Substanz durch ein Gerät aus der Gruppe, bestehend aus einem Ionen-Mobilitäts-Nachweis-Analysator, einem Massenspektrometer und einem Chromatographen.

5. Verfahren nach irgendeinem der Ansprüche 1 bis 4, einschließend ein Verwenden eines Ionen-Mobilitäts-Detektors zum Analysieren der zu analysierenden Substanz auf die Gegenwart der Verbindung, was anzeigt, dass das Gehäuse ein Leck aufweist, oder auf die Abwesenheit der Verbindung, was anzeigt, dass das Gehäuse hermetisch dicht ist, vorzugsweise einschließend ein Analysieren der zu analysierenden Substanz unter Verwendung eines Ionen-Mobilitäts-Nachweis-Analysators bei Umgebungsdruck oder einschließend ein Analysieren der zu analysierenden Substanz unter Verwendung eines Ionen-Mobilitäts-Nachweis-Analysators unter Vakuum, noch mehr bevorzugt einschließend ein Verwenden des Ionen-Mobilitäts-Nachweis-Analysators in einem kontinuierlichen Lauf oder einem Batch-Lauf.

6. Verfahren nach irgendeinem der Ansprüche 1 bis 5, worin das hermetisch dichte Gehäuse (110) entweder eine elektrochemische Zelle oder einen Kondensator umfasst und weiter einschließend ein Anbringen der hermetisch dichten elektrochemischen Zelle oder des Kondensators in eine implantierbare medizinische Vorrichtung als deren Energiequelle.

7. Verfahren nach irgendeinem der Ansprüche 1 bis 6, einschließend ein Erhitzen des Gehäuses (110) auf eine Temperatur bis zu 125 °C, wenn man den gasförmigen Strom an ihm vorbei fließen lässt.

## Revendications

1. Procédé pour déterminer l'intégrité d'étanchéité d'une enceinte (110), comprenant les étapes :
a) procurer, dans une première chambre dimensionnée pour contenir l'enceinte (110), l'enceinte (110) comprenant au moins une première partie fixée à une deuxième partie avec un liquide contenu dans au moins une portion d'un volume renfermé de l'enceinte (110) ;
b) procurer le liquide comprenant au moins un composé constitutif possédant une pression de vapeur à 25 °C qui est supérieure à 13,3 Pa ;
c) faire s'écouler un courant gazeux d'air purifié ou d'air ambiant devant l'enceinte (110) dans la première chambre pour ainsi obtenir un analyte ; et
d) analyser l'analyte pour détecter la présence du composé indiquant que l'enceinte (110) fuit ou pour détecter l'absence du composé indiquant que l'enceinte (110) est hermétiquement fermée.

2. Procédé selon la revendication 1, dans lequel le composé est choisi parmi le groupe constitué par l'acide acétique, l'éthylène glycol, le diéthylène glycol, le propylène glycol, le dipropylène glycol, le glycérol, le 2-méthyl-1,3-propanediol, le tétraéthylène glycol, les polyéthylène glycols, les polypropylène glycols, des copolymères de polyéthylène-polypropylène glycol, l'éther méthylique d'éthylène glycol, l'éther éthylique d'éthylène glycol, l'éther méthylique de diéthylène glycol, l'éther éthylique de diéthylène glycol, l'éther méthylique de dipropylène glycol, l'éther méthylique de tripropylène glycol, l'éther diméthylique d'éthylène glycol, l'éther diméthylique de triéthylène glycol, le N-éthylformamide, le N-méthylformamide, le N,N-diméthylformamide, le N-méthylacétamide, le N,N-diméthylacétamide, la N-méthylpyrrolidone, le diméthylacétamide, le lactate d'éthyle, le diacétate d'éthylène, l'acétonitrile, le propionitrile, le méthoxypropionitrile, la γ-butyrolactone, la y-valérolactone, le carbonate de diméthyle, le carbonate de diéthyle, le carbonate de dipropyle, le carbonate d'éthylène, le carbonate de propylène, le carbonate de butylène, le carbonate d'éthylméthyle, le carbonate de méthylpropyle, le carbonate d'éthylpropyle, le carbonate d'isopropylméthyle, le sulfolane, le 3-méthylsulfolane, le diméthylsulfoxyde, le diméthylformamide, l'acétate de diméthyle, le diméthylsulfolane, le tétrahydrofuranne, l'acétate de méthyle, le diglyme, le triglyme, le tétraglyme, l'éther diisopropylique, le 1,2-diméthoxyéthane, le 1,2-diéthoxyéthane, le 1-éthoxy-2-méthoxyéthane, le 2-méthyltétrahydrofuranne, la 3-méthyl-2-oxazolidinone, le benzène, le cumène, l'éthylbenzène, l'éthyldiglyme, l'éthylmonoglyme, le fluorotrichlorométhane, le chlorure de méthylène, la propylsulfone, le pseudocumène, le tétraéthylorthosilicate, le toluène, le m-xylène, le o-xylène, l'acétate d'ammonium, le phosphate d'ammonium, le borate d'ammonium, l'acide propionique, l'acide butyrique, l'acide méthylbutyrique, l'acide isobutyrique, l'acide triméthylacétique, et leurs mélanges.

3. Procédé selon la revendication 1 ou 2, dans lequel le liquide comprend un électrolyte d'une cellule électrochimique ou d'un condensateur, de préférence dans lequel le liquide comprend un électrolyte et le composé est l'acide acétique.

4. Procédé selon l'une quelconque des revendications 1 à 3, englobant l'analyse de l'analyte via un élément choisi parmi le groupe constitué par un analyseur de la détection de la mobilité ionique, un spectromètre de masse et un chromatographe.

5. Procédé selon l'une quelconque des revendications 1 à 4, englobant l'utilisation d'un détecteur de la mobilité ionique pour analyser l'analyte afin de détecter la présence du composé indiquant une fuite dans l'enceinte ou afin de détecter l'absence du composé indiquant une fermeture hermétique de l'enceinte, de préférence englobant l'analyse de l'analyte en utilisant un analyseur de la détection de la mobilité ionique sous la pression ambiante, ou englobant l'analyse de l'analyte en utilisant un analyseur de la détection de la mobilité ionique sous vide, de manière plus préférée englobant l'utilisation de l'analyse de la détection de la mobilité ionique dans un mode en continu ou dans un mode en discontinu.

6. Procédé selon l'une quelconque des revendications 1 à 5, dans lequel l'enceinte hermétiquement fermée (110) comprend, soit une cellule électrochimique, soit un condensateur, et englobant en outre le fait d'incorporer le condensateur ou la cellule électrochimique hermétiquement fermée dans un dispositif médical implantable pour faire office de source d'alimentation pour celui-ci ou celle-ci.

7. Procédé selon l'une quelconque des revendications 1 à 6, englobant le fait de chauffer l'enceinte (110) à une température allant jusqu'à 125 °C lorsque le courant gazeux s'écoule en passant devant elle.
